# FASCICULE DE BREVET EUROPEEN

(11) **EP 0 846 688 B1**
(45) Date de publication et mention de la délivrance du brevet: **16.02.2000**
(21) Numéro de dépôt: 97402891.2
(22) Date de dépôt: 02.12.1997
(51) Int. Cl.: C07D 233/06, A61K 31/415, C07D 233/18, C07D 233/20, C07D 233/22, C07D 233/90, C07D 401/04, C07D 403/04, C07D 417/10, C07D 233/24

(54) **Nouveaux dérivés de l'imidazoline ayant une affinité pour les récepteurs aux imidazolines**
Neue Imidazolinderivate mit Imidazolin-Rezeptor Affinität
New imidazoline derivatives having affinity for the imidazoline receptor

(30) Priorité: 04.12.1996 FR 9614844
(43) Date de publication de la demande: 10.06.1998
(73) Titulaire: ADIR ET COMPAGNIE, 92415 Courbevoie Cédex (FR)
(72) Inventeur: Payard, Marc, 31130 Balma (FR); Danoun, Saadia, 31400 Toulouse (FR); Baziard-Mouysset, Geneviève, 31068 Toulouse (FR); Anastassiadou, Maria, 31068 Toulouse (FR); Caignard, Daniel-Henri, 78230 Le Pecq (FR); Renard, Pierre, 78000 Versailles (FR); Manechez, Dominique, 59290 Wasquehal (FR); Scalbert, Elizabeth, 92100 Boulogne (FR); Rettori, Marie-Claire, 92400 Courbevoie (FR)

(56) Documents cités:
- US-A- 5 574 059
- KLEM R E ET AL: "Dehydrogenation of 2-aryl-2-imidazolines with selenium" J. HETEROCYCL. CHEM. , vol. 7, no. 2, avril 1970, pages 403-404, XP002058798
- CHEMICAL ABSTRACTS, vol. 089, no. 5, 31 juillet 1978 Columbus, Ohio, US; abstract no. 042718, PISKOV V B ET AL: "Synthesis and some physicochemical properties of benzamidines and their analogs" page 566; colonne 1; XP002058800 & ZH. ORG. KHIM. , vol. 14, no. 4, 1978, pages 820-834,
- HOULIHAN W J ET AL: "Antitumor Activity of 5-Aryl-2,3-dihydroimidazo[2,1-a]isoquinoli nes" J. MED. CHEM. , vol. 38, no. 2, 20 janvier 1995, pages 234-240, XP002058799
- D.J. NUTT ET AL.: "Functional Studies of Specific Imidazoline-2 Receptor Ligands" ANNALS OF THE NEW YORK ACADEMY OF SCIENCES, vol. 763, 1995, NEW YORK, pages 125-139, XP002038401

## Description

L'invention concerne de nouveaux dérivés de l'imidazoline, leur procédé de préparation ainsi que les compositions pharmaceutiques qui les contiennent.

Sur le plan des structures chimiques, la littérature fournit de très nombreux exemples de dérivés de l'imidazoline.

Par exemple les brevets JP-07010871 et EP-501074 revendiquent des composés comportant un motif imidazoline, proches de ceux de la présente invention, et utiles comme agents de réticulation de résines époxy.
Les travaux de E. Uhlig et coll. (*Z. Anorg. Allg. Chem., 534*, (1986), 188-198) présentent des dérivés de l'imidazoline agissant comme complexants d'ions cuivriques.

Lorsqu'ils sont utilisés en thérapeutique, les dérivés de l'imidazoline sont également très largement décrits.

Par exemple, le brevet JP-60209571 présente des tétrahydropyrimidines et imidazolines à propriétés analgésiques.

Par ailleurs de très nombreuses publications (J.N. Sengupta et coll., *Naunyn-Schniedeberg's Arch. Pharmacol., 335* (4), (1987), 391-396 ; H. Fuder et coll., *Pharmacol. Adrenoreceptors,* (1984), 335-336 ; R.R. Ruffolo, *Eur. J. Pharmacol., 157* (2-3), (1988), 235-239) présentent des études pharmacologiques de dérivés d'imidazoline, ligands des récepteurs adrénergiques.

La présente invention a pour objet de nouveaux dérivés de l'imidazoline de structure originale présentant une très haute affinité pour les récepteurs aux imidazolines.

De tels dérivés sont bien évidemment intéressants dans le traitement des maladies cardiovasculaires comme l'hypertension. Ainsi, on connaît la clonidine utilisée largement depuis de nombreuses années dans le traitement de l'hypertension artérielle.

II est également connu que les récepteurs aux imidazolines interviennent dans la stimulation de la libération d'insuline par les cellules β du pancréas (Schutz et coll., *Naunyn-Schniedeberg's Arch. Pharmacol.,* (1989), *340* (6), 712-714).

On a également rapporté l'intérêt de ligands aux récepteurs des imidazolines dans le traitement des désordres psychiatriques et neurologiques comme la dépression, la maladie de Parkinson et l'anorexie (D.J. NUTT et coll., *Annals New York Academy of Sciences,* (1995), 125-139).

Toutefois, la grande majorité des dérivés imidazoliniques connus à ce jour ont, outre leur affinité pour les récepteur imidazoliniques, une grande affinité pour les récepteurs adrenergiques ce qui entraîne l'apparition d'une forte composante cardiovasculaire.

La demanderesse a présentement découvert de nouveaux dérivés à structure imidazolinique ligands puissants des récepteurs imidazoliniques mais dépourvus d'affinité pour les récepteurs adrenergiques.

Ainsi les composés de l'invention trouvent une utilisation tout particulièrement intéressante en thérapeutique pour le traitement des pathologies liées aux récepteurs aux imidazolines de par leur haute affinité pour ces récepteurs, tout en étant dépourvus des effets secondaires d'origine centrale, du fait de leur très faible affinité pour les récepteurs adrénergiques. De ce fait les dérivés de l'invention sont intéressants dans le traitement des maladies cardiovasculaires, de l'hypertension mais également dans le traitement de la maladie diabétique ainsi que dans le traitement des désordres psychiatriques et neurologiques comme la dépression, la maladie de Parkinson et l'anorexie ce qui n'était pas le cas des imidazolines connues dans l'art antérieur.

Plus spécifiquement, la présente invention concerne les composés de formule (I) : 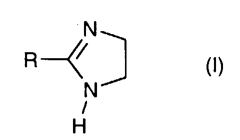 dans laquelle R représente :
- soit un radical phényle de formule (α) : 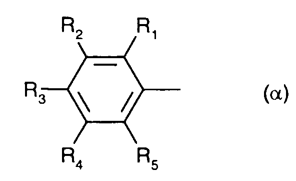 dans laquelle R₁ représente :
   ◆ soit un radical (C₁-C₆) alkyle, et dans ce cas :
      ◇ ou bien R₂ représente un atome d'hydrogène, de même que R₃ et R₅, et dans ce cas R₄ représente un atome d'halogène différent d'un chlore,
      ◇ ou bien R₂ représente un atome d'hydrogène, de même que R₄ et R₅, et dans ce cas R₃ représente un atome d'halogène différent d'un chlore,
      ◇ ou bien R₂ représente le radical cyano, et R₃, R₄ et R₅ représentent chacun un atome d'hydrogène,
   ◆ soit un atome d'halogène, et dans ce cas :
      ◇ ou bien R₃ représentent lui aussi un atome d'halogène différent de R₁, et R₂, R₄ et R₅ représentent chacun un atome d'hydrogène,
      ◇ ou bien R₃ représente un atome d'hydrogène ainsi que R₂ et R₅, et R₄ représente alors un radical (C₁-C₆) alkyle,
   ◆ soit un atome d'hydrogène, et dans ce cas :
      ◇ ou bien R₂, R₃ et R₄ représentent simultanément un atome d'hydrogène et R₅ représente un groupement phényle,
      ◇ ou bien R₂ et R₃ représentent simultanément un atome d'halogène différent l'un de l'autre, et R₄ et R₅ chacun un atome d'hydrogène,
      ◇ ou bien R₂ représente un atome d'halogène (différent d'un chlore et d'un fluor) ou un groupement (C₂-C₆) alkyle ou (C₁-C₆) alkylcarbonyle, et R₃, R₄, R₅ représentent chacun un atome d'hydrogène,
      ◇ ou bien R₂ représente un atome d'hydrogène, de même que R₄ et R₅ et dans ce cas, R₃ est choisi parmi un radical éthyle, n-propyle, n-butyle, trifluorométhyle, (C₁-C₆) alkylthio, trifluorométhoxy, phényle, phénoxy, (C₁-C₆) acylamino, aminosulfonyle, aminosulfonyle substitué sur l'atome d'azote par un ou deux groupements (C₁-C₆) alkyle,
      ◇ ou bien R₂ représente un groupement nitro, R₃ représente un groupement hydroxy, R₄ représente un atome d'halogène et R₅ représente un atome d'hydrogène,
      ◇ ou bien R₃ et R₅ représentent chacun un atome d'hydrogène et R₂ et R₄ représentent chacun un atome d'halogène, sans pouvoir représenter simultanément deux atomes de chlore,
- soit un radical naphtyle de formule (β): 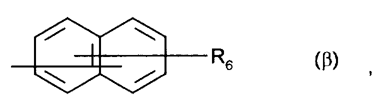 dans laquelle R₆ représente un atome d'halogène, un groupement (C₁-C₆) alkyle, ou méthoxy,
- soit un radical choisi parmi les radicaux
   - indol-5-yle 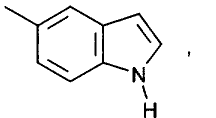
   - 1-phényl-1-cyclohexylméthyle,
   - cycloheptyle,
   - 4-(benzothiazol-2-yl)benzyle, et
   - le radical 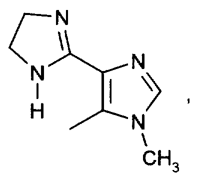
leurs isomères optiques, ainsi que leurs sels d'addition à un acide, pharmaceutiquement acceptables,
étant entendu que, par radical (C₁-C₆) alkyle, (C₂-C₆) alkyle et radical (C₁-C₆) alkylthio, on entend aussi bien les radicaux linéaires que les radicaux ramifiés.

Parmi les acides pharmaceutiquement acceptables que l'on peut utiliser pour former un sel d'addition avec les composés de l'invention, on peut citer, à titre d'exemples et de façon non limitative, les acides chlorhydrique, sulfurique, phosphorique, tartrique, malique, maléique, fumarique, oxalique, méthanesulfonique, éthanesulfonique, camphorique, et citrique.

Les halogènes présents dans les composés de formule générale (I) sont choisis parmi le brome, le chlore, le fluor et l'iode.

L'invention concerne préférentiellement les composés de formules : 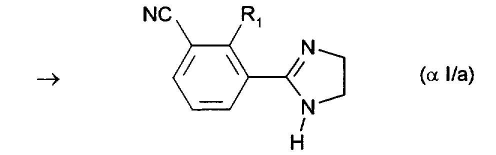 dans laquelle R₁ représente un groupement (C₁-C₆) alkyle linéaire ou ramifié, 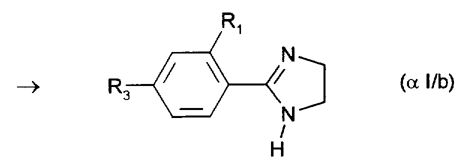 dans laquelle R₁ représente un atome d'halogène ou un groupement (C₁-C₆) alkyle, linéaire ou ramifié, et R₃ différent de R₁ représente un atome d'halogène (R₃ étant différent d'un atome de chlore lorsque R₁ représente un groupement (C₁-C₆) alkyle linéaire ou ramifié), 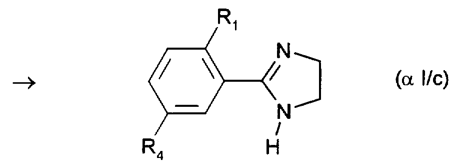 dans laquelle R₁ représente un atome d'halogène et R₄ représente un radical (C₁-C₆) alkyle, linéaire ou ramifié, 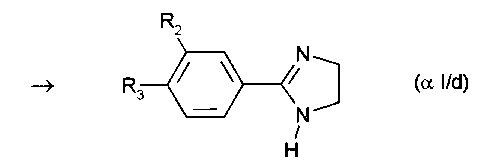 dans laquelle R₂ et R₃ représentent chacun un atome d'halogène différent l'un de l'autre, 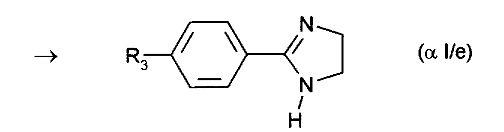 dans laquelle R₃ représente un radical choisi parmi un radical éthyle, n-propyle, n-butyle, trifluorométhyle, (C₁-C₆) alkylthio, trifluorométhoxy, phényle, phénoxy, (C₁-C₆) acylamino, aminosulfonyle, aminosulfonyle substitué sur l'atome d'azote par un ou deux groupements (C₁-C₆) alkyle linéaire ou ramifié), 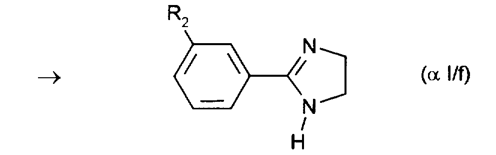 dans laquelle R₂ représente un atome d'halogène différent d'un chlore et d'un fluor, ou un groupement (C₂-C₆) alkyle linéaire ou ramifié ou (C₁-C₆) alkylcarbonyle linéaire ou ramifié.

Plus particulièrement, l'invention concerne les composés de formule (I) pour lesquels R est choisi parmi les radicaux :
- 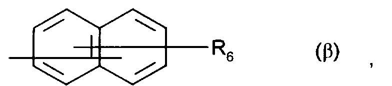 dans lequel R₆ représente un atme d'halogène, un groupement (C₁-C₆) alkyle linéaire ou ramifié, ou méthoxy,
   par exemple R représente un groupement 2-méthoxy-1-naphtyl,
- 5-indolyle,
- cycloheptyle.

L'invention concerne également le procédé de préparation des composés de formule (I), caractérisé en ce que l'on opère une condensation d'un nitrile de formule (II) :

R-C≡N (II)

dans laquelle R est tel que défini précédemment,
avec l'éthylène diamine, en présence d'une quantité catalytique de pentasulfure de phosphore,
le composé brut ainsi obtenu pouvant être, si on le désire :
- purifié suivant une ou plusieurs méthodes de purification choisies parmi la cristallisation, la chromatographie sur gel de silice, l'extraction, la filtration et le passage sur charbon ou sur résine,
- séparé, le cas échéant, sous forme pure ou sous forme de mélange, en ses éventuels isomères optiques, selon des techniques classiques de séparation,
- et/ou transformé par un acide, en sels pharmaceutiquement acceptables.

Les matières premières utilisées dans le procédé de préparation des composés de formule (I) sont soit commerciales soit aisément accessibles à l'homme du métier.

Les composés de formule (I) possèdent des propriétés pharmacologiques très intéressantes pour le clinicien et le médecin.

Les composés de l'invention et les compositions pharmaceutiques qui les contiennent s'avèrent être de puissants ligands des récepteurs aux imidazolines I₁ et/ou I₂.

Les récepteurs aux imidazolines sont également impliqués dans l'anémie, notamment l'anémie à hématies falciformes, et la prolifération cancéreuse.

Par ailleurs, les études pharmacologiques des composés de l'invention ont démontré une absence totale de toxicité outre leur très haute affinité pour les récepteurs aux imidazolines déjà mentionnée.

Ceci permet d'établir que les composés de l'invention et les compositions pharmaceutiques qui les contiennent sont utiles dans le traitement des pathologies liées au système nerveux central et notamment de la dépression, la maladie de Parkison, l'anorexie, des pathologies cardio-vasculaires et notamment de l'hypertension, ainsi que dans le traitement du diabète, de l'obésité, de l'anémie, notamment l'anémie à hématies calciformes et du cancer.

La présente invention a également pour objet les compositions pharmaceutiques contenant les produits de formule (I) ou le cas échéant un de leurs sels d'addition à un acide, pharmaceutiquement acceptables en combinaison avec un ou plusieurs excipients pharmaceutiquement acceptables.

Parmi les compositions pharmaceutiques selon l'invention, on pourra citer, plus particulièrement celles qui conviennent pour l'administration orale, parentérale, nasale, per- ou trans-cutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, les ampoules buvables ou injectables et les aérosols.

La posologie varie selon le sexe, l'âge et le poids du patient, la voie d'administration, la nature de l'indication thérapeutique, ou des traitements éventuellement associés et s'échelonne entre 0,1 mg et 100 mg par 24 heures en 1 ou 2 prises, plus particulièrement entre 1 et 10 mg, par exemple entre 1 et 2 mg.

Les exemples suivants illustrent l'invention, mais ne la limitent en aucune façon.

### Exemples 1 à 30 :

### Mode opératoire général :

Un mélange constitué de 25 ml d'éthylène diamine, de 0,01 moles à 0,02 moles d'un nitrile et d'une quantité catalytique (0,5 g environ) de pentasulfure de phosphore est chauffé à reflux, sous agitation pendant 4 à 8 heures. La disparition du nitrile est suivie par chromatographie sur couche mince. Le mélange refroidi est versé dans 50 ml d'eau froide. L'ensemble est alors extrait par deux fois 50 ml de dichlorométhane. Après évaporation de la fraction organique, le résidu est recristallisé dans le cyclohexane.

En procédant comme il est décrit dans le mode opératoire général et en utilisant le nitrile approprié, les composés des exemples suivants sont obtenus :

### Exemple 1 : 2-(4-Biphényl) Δ-2 imidazoline

Rendement : 91%
Point de fusion : 200°C

### Exemple 2 : 2-(4-Trifluorométhoxyphényl) Δ-2 imidazoline

Rendement : 77%
Point de fusion : 150°C

### Exemple 3 : 2-(5-Indolyl) Δ-2 imidazoline

Rendement : 60%
Point de fusion : 181°C

### Exemple 4 : 2-(1-Cyclohexyl-1-phénylméthyl) Δ-2 imidazoline

Rendement : 41%
Point de fusion : 178°C

### Exemple 5 : 2-[4-(Benzothiazol-2-yl)benzyl] Δ-2 imidazoline

Rendement : 52%
Point de fusion : 157°C

### Exemple 6 : 2-(6-Méthoxy-2-naphtyl) Δ-2 imidazoline

Rendement : 61%
Point de fusion : 155°C

### Exemple 7 : 2-Cycloheptyl Δ-2 imidazoline

Rendement : 77%
Point de fusion : 255°C

### Exemple 8 : 2-(4-Ethylphényl) Δ-2 imidazoline

Rendement : 98%
Point de fusion : 135°C

### Exemple 9 : 1-Méthyl-4,5-bis(Δ-2 imidazolin-2-yl)imidazole

Rendement : 84%
Point de fusion : 162°C

### Exemple 10 : 2-(4-n-Propylphényl) Δ-2 imidazoline

Rendement : 70%
Point de fusion : 126°C

### Exemple 11 : 2-(4-n-Butylphényl) Δ-2 imidazoline

Rendement : 61%
Point de fusion : 98°C

### Exemple 12 : 2-(3-Cyano-2-méthylphényl) Δimidazoline

Rendement : 78%
Point de fusion : 144°C

### Exemple 13 : 2-(4-Phénoxyphényl) Δ-2 imidazoline

Rendement : 56%
Point de fusion : 129°C

### Exemple 14 : 2-(3-Chloro-4-fluorophényl) Δ-2 imidazoline

Rendement : 41%
Point de fusion : 109°C

### Exemple 15 : 2-(2-Chloro-4-fluorophényl) Δ-2 imidazoline

Rendement : 40%
¹H RMN δ (ppm) : 2,97 et 3,16 (2m, 4H, 2CH₂) ; 4,93 (s, 1H, NH) ; 6,46 (dd, 1H, *J* = 2,37 et 8,71 Hz, H₅) 6,59 (d, 1 H, J = 2,37 Hz, H₃); 7,35 (d, 1*H*, J = 8,71 Hz, H6).

### Exemple 16 : 2-(2-Fluoro-5-méthylphényl) Δ-2 imidazoline

Rendement : 40%
Point de fusion : 85°C

### Exemple 17 : 2-(4-Ethylthiophényl) Δ-2 imidazoline

### Exemple 18 : 2-(4-Méthylthiophényl) Δ-2 imidazoline

Rendement : 72%
Point de fusion : 158°C

### Exemple 19 : 2-(2-Méthoxy-1-naphtyl) Δ-2 imidazoline

Rendement : 70%
Point de fusion : 157°C

### Exemple 20 : 2-(4-Trifluorométhylphényl) Δ-2 imidazoline

Rendement : 81%
Point de fusion : 180°C

### Exemple 21 : 2-(3-Ethylphényl) Δ-2 imidazoline

### Exemple 22 : 2-(2-Phénylphényl) Δ-2 imidazoline

### Exemple 23 : 2-(5-Fluoro-2-méthylphényl) Δ-2 imidazoline

### Exemple 24 : 2-(4-Hydroxy-5-iodo-3-nitrophényl) Δ-2 imidazoline

### Exemple 25 :2-(4-Aminosulfonylphényl) Δ-2 imidazoline

### Exemple 26 : 2-(4-Acétylaminophényl) Δ-2 imidazoline

### Exemple 27 : 2-(4-Méthyl-1-naphtyl) Δ-2 imidazoline

### Exemple 28 : 2-(4-Fluoro-1-naphtyl) Δ-2 imidazoline

### Exemple 29 : 2-(4-Bromo-2-méthylphényl) Δ-2 imidazoline

### Exemple 30 : 2-(3,5-Difluorophényl) Δ-2 imidazoline

### ETUDE PHARMACOLOGIQUE

### Exemple A : Profil de liaison aux récepteurs imidazolines I₁ et I₂

### Objectif :

Mesurer, in vitro, l'affinité de liaison des composés de l'invention aux récepteurs I₁ et I₂, en déterminant la capacité de ces composés à déplacer les radioligands spécifiques des récepteurs aux imidazolines I₁ et I₂.

### Protocole :

Le tableau suivant indique le radioligand utilisé pour marquer le récepteur, le produit et la concentration retenue pour déterminer la fraction non spécifique et le tissu choisi.

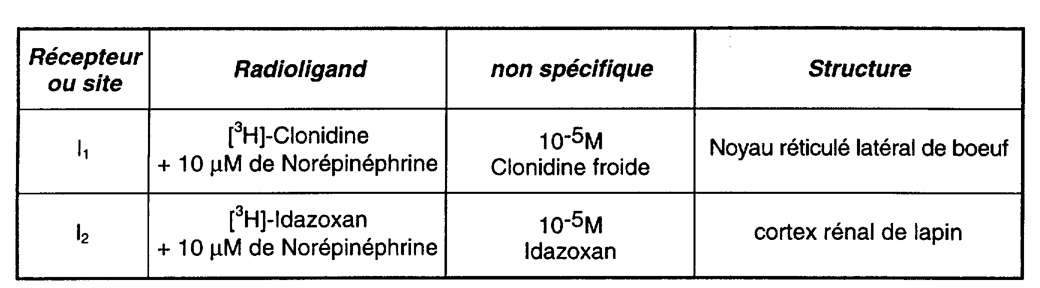

### Résultats :

Les résultats obtenus *in vitro* sur les récepteurs centraux ou périphériques et avec nos conditions expérimentales, montrent que les composés de l'invention présentent une très haute affinité pour les sites I₁ et/ou I₂ du cortex rénal de lapin avec des Kᵢ variant de quelques nM à quelques centaines de nM.

### Exemple B : Profil de liaison aux récepteurs centraux adrénergiques α₁ et α₂

### Objectif :

Mesurer, in vitro, l'affinité de liaison des composés de l'invention aux récepteurs centraux α₁ et α₂, en déterminant la capacité de ce produit à déplacer les radioligands spécifiques de ces récepteurs.

### Protocole :

Le tableau suivant indique le radioligand utilisé pour marquer le récepteur, le produit et la concentration retenue pour déterminer la fraction non spécifique et le tissu choisi.

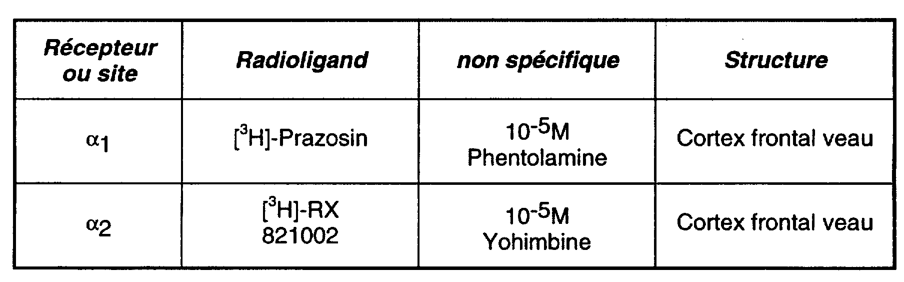

### Résultats :

Les résultats obtenus, *in vitro* sur les récepteurs adrénergiques avec nos conditions expérimentales, montrent que les composés de l'invention n'ont qu'une très basse affinité pour les récepteurs α₁-adrénergiques (Kᵢ > 7 µM) et α₂-adrénergiques (Kᵢ > 10 µM).

### Exemple C : Test du désespoir comportemental

Les souris utilisées pour ce test sont placées dans un cylindre rempli d'eau d'où elles ne peuvent échapper. Après quelques efforts pour s'en sortir, les animaux se résignent et s'immobilisent, ne faisant plus que les mouvements nécessaires pour maintenir la tête hors de l'eau. Les animaux, par groupe de dix, sont ainsi placés dans le cylindre pendant 6 minutes, et la durée d'immobilité est mesurée pendant les 4 dernières minutes.
La durée de l'immobilité permet de caractériser l'activité antidépressive des composés testés. Ainsi, des antidépresseurs tels que l'imipramine ou la désipramine diminuent cette durée d'immobilité.
Les composés de l'invention ont montré une activité comparable à celle de l'imipramine et la désipramine, le temps d'immobilité mesuré étant du même ordre que celui obtenu avec les produits de référence.

### Exemple D : Mesure de l'affinité pour la monoamine oxydase

### In vitro

Les tests de liaison au site imidazolinique 12 ainsi que l'affinité pour la monoamine oxydase sont réalisés selon le protocole décrit par C. Carpéné (Annals. N.Y. Acad. Sci., 1995, *763*, p. 380).
Le radioligand de référence utilisé est le BFI tritié. Des expériences de liaison compétitive sont réalisées avec les composés de l'invention dans le but de mettre en évidence leur capacité à déplacer le radioligand de référence.

### Ex vivo

Les animaux utilisés sont des rats Zücker génétiquement obèses, qui sont soumis à un traitement sub-chronique à l'aide des composés à tester. A l'issu de ce test, la liaison aux sites imidazoliniques 12 ainsi que l'activité de la monoamine oxydase sont mesurées après extraction du tissu adipeux ex vivo selon une méthode décrite par C. Carpéné (J. lipids. Res., 1990, *31*, p. 811).

### Résultats

II apparaît que les composés de l'invention posèdent une affinité élevée pour les sites de liaison imidazoliniques 12, de l'ordre de 1 à 100 nM, et un effet inhibiteur sur la monoamine oxydase dans les adipocytes en se liant à l'enzyme avec une affinité de l'ordre de 10⁻⁶M.

### Exemple E : Activité hypoglycémiante

L'activité hypoglycémiante des dérivés de l'invention a été recherchée sur des rats mâles Witsar d'environ 250 g âgés de trois mois. Un diabète expérimental est obtenu par injection *iv* d'une faible dose de streptozotocine dissoute dans un tampon citrate (171) sous anesthésie au chlorhydrate de Kétamine (75 mg.kg⁻¹, lp). Ces rats sont appelés "STZ", et les rats normaux ont reçu une injection de tampon citrate dans les mêmes conditions.
L'homéostasie a été évaluée par un test de tolérance au glucose, réalisé deux semaines après injection de streptozotocine.
. ***Test de tolérance au glucose par voie intraveineuse (IVGTT)***
   Le glucose est dissout dans une solution aqueuse de NaCI à 0,9 % et administré par la voie de la veine saphène à des rats anesthésiés au pentobarbital (60 mg.kg⁻¹, *IP*). Les échantillons de sang sont collectés séquentiellement par les vaisseaux de la queue avant et 5, 10, 15, 20 et 30 minutes après l'injection du glucose. Ils sont ensuite centrifugés et le plasma est séparé. La concentration en glucose plasmatique est déterminée immédiatement sur un aliquot de 10 µl et le plasma restant est conservé à -20°C.
   Une injection unique en *IP* du produit à tester est effectuée à des rats anesthésiés au pentobarbital, 20 minutes avant l'IVGTT.
. ***Test de tolérance au glucose par voie orale (OGTT)***
   Le glucose est administré *per os* (2g.kg⁻¹) à des rats vigiles. Les échantillons de sang sont collectés avant et 10, 20, 30, 40, 60, 90 et 120 minutes après l'administration du glucose. Le traitement des échantillons sanguins est identique à celui décrit précédemment.
   Le produit à tester est administré *per os* 30 minutes avant l'OGTT.
. ***Méthodes analytiques***
   La concentration en glucose plasmatique est déterminée par utilisation d'un analyseur de glucose (Beckman Inc., Fullerton, CA). La tolérance au glucose est mesurée par rapport à deux paramètres : ΔG et K.
   ΔG représente l'augmentation de la glycémie au dessus de la ligne de base, intégrée sur une période de 30 minutes (IVGTT) ou de 120 minutes (OGTT), après surcharge en glucose.
   K est la vitesse de disparition du glucose entre 5 et 30 minutes (IVGTT), après administration du glucose. Le coefficient K est calculé uniquement pendant l'IVGTT.
   Il apparaît que les composés de l'invention ont une activité comparable à celle du gliclazide, et présentent l'avantage de ne pas induire la même hypoglycémie basale.

### Exemple F : Etude de la toxicité aigüe

La toxicité aigüe a été appréciée après administration orale à des lots de 8 souris (26 ± 2 gammes) de doses croissantes de produit à étudier. Les animaux ont été observés à intervalles réguliers au cours de la première journée et quotidiennement pendant les deux semaines suivant le traitement.
Il apparaît que les composés de l'invention sont très peu toxiques.

### Exemple G : Composition pharmaceutique : Comprimés

Formule de préparation pour 1000 comprimés dosés à 1 mg de 2-(4-éthylphényl) Δ-2 imidazoline :

| | |
|---|---|
| 2-(4-Ethylphényl) Δ-2 imidazoline | 1 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

1. Composés de formule (I) : 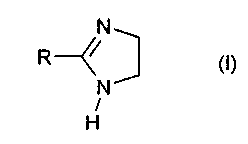 dans laquelle R représente :
• soit un radical phényle de formule (α) : 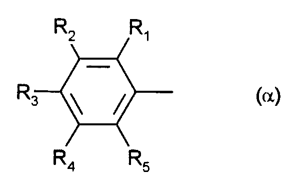 dans laquelle R₁ représente :
◆ soit un radical (C₁-C₆) alkyle, et dans ce cas :
◇ ou bien R₂ représente un atome d'hydrogène, de même que R₃ et R₅, et dans ce cas R₄ représente un atome d'halogène différent d'un chlore,
◇ ou bien R₂ représente un atome d'hydrogène, de même que R₄ et R₅, et dans ce cas R₃ représente un atome d'halogène différent d'un chlore,
◇ ou bien R₂ représente le radical cyano, et R₃, R₄ et R₅ représentent chacun un atome d'hydrogène,
◆ soit un atome d'halogène, et dans ce cas :
◇ ou bien R₃ représente lui aussi un atome d'halogène différent de R₁, et R₂, R₄ et R₅ représentent chacun un atome d'hydrogène,
◇ ou bien R₃ représente un atome d'hydrogène ainsi que R₂ et R₅, et R₄ représente alors un radical (C₁-C₆) alkyle,
◆ soit un atome d'hydrogène, et dans ce cas :
◇ ou bien R₂, R₃ et R₄ représentent simultanément un atome d'hydrogène et R₅ représente un groupement phényle,
◇ ou bien R₂ et R₃ représentent simultanément un atome d'halogène différent l'un de l'autre, et R₄ et R₅ chacun un atome d'hydrogène,
◇ ou bien R₂ représente un atome d'halogène différent d'un chlore et d'un fluor ou un groupement (C₂-C₆) alkyle ou (C₁-C₆) alkylcarbonyle, et R₃, R₄, R₅ représentent chacun un atome d'hydrogène,
◇ ou bien R₂ représente un atome d'hydrogène, de même que R₄ et R₅ et dans ce cas, R₃ est choisi parmi un radical éthyle, n-propyle, n-butyle, trifluorométhyle, (C₁-C₆) alkylthio, trifluorométhoxy, phényle, phénoxy, (C₁-C₆) acylamino, aminosulfonyle, aminosulfonyle substitué sur l'atome d'azote par un ou deux groupements (C₁-C₆) alkyle,
◇ ou bien R₂ représente un groupement nitro, R₃ représente un groupement hydroxy, R₄ représente un atome d'halogène et R₅ représente un atome d'hydrogène,
◇ ou bien R₃ et R₅ représentent chacun un atome d'hydrogène et R₂ et R₄ représentent chacun un atome d'halogène, sans pouvoir représenter simultanément deux atomes de chlore,
• soit un radical naphtyle de formule (β): 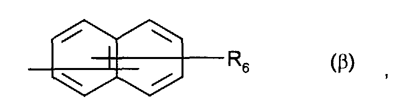 dans laquelle R₆ représente un atome d'halogène, un groupement (C₁-C₆) alkyle, ou méthoxy,
• soit un radical choisi parmi les radicaux
- indol-5-yle 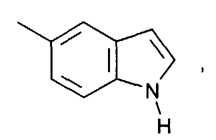
- 1-phényl-1-cyclohexylméthyle,
- cycloheptyle,
- 4-(benzothiazol-2-yl)benzyle, et
- le radical 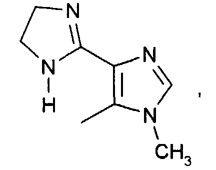
leurs isomères optiques, ainsi que leurs sels d'addition à un acide, pharmaceutiquement acceptables,
étant entendu que, par radical (C₁-C₆) alkyle, (C₂-C₆) alkyle et radical (C₁-C₆) alkylthio, on entend aussi bien les radicaux linéaires que les radicaux ramifiés.

2. Composés selon la revendication 1 de formule (α I/a): 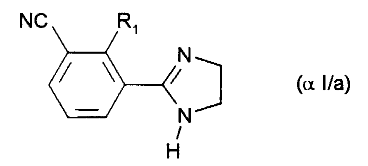 dans laquelle R₁ représente un groupement (C₁-C₆) alkyle linéaire ou ramifié,
leurs isomères optiques, ainsi que leurs sels d'addition à un acide pharmaceutiquement acceptable.

3. Composés selon la revendication 1 de formule (α I/b): 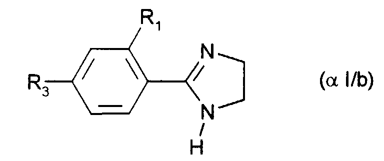 dans laquelle R₁ représente un atome d'halogène ou un groupement (C₁-C₆) alkyle, linéaire ou ramifié, et R₃ différent de R₁ représente un atome d'halogène (R₃ étant différent d'un atome de chlore lorsque R₁ représente un groupement (C₁-C₆) alkyle linéaire ou ramifié),
leurs isomères optiques, ainsi que leurs sels d'addition à un acide pharmaceutiquement acceptable.

4. Composés selon la revendication 1 de formule (α I/c): 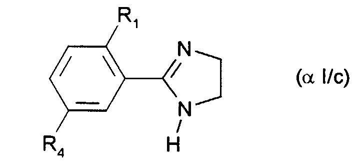 dans laquelle R₁ représente un atome d'halogène et R₄ représente un radical (C₁-C₆) alkyle, linéaire ou ramifié,
leurs isomères optiques, ainsi que leurs sels d'addition à un acide pharmaceutiquement acceptable.

5. Composés selon la revendication 1 de formule (α I/d) : 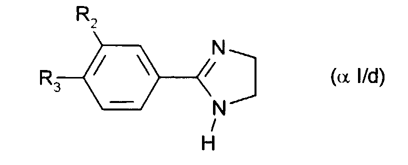 dans laquelle R₂ et R₃ représentent chacun un atome d'halogène différent l'un de l'autre,
ainsi que leurs sels d'addition à un acide pharmaceutiquement acceptable.

6. Composés selon la revendication 1 de formule (α I/e) : 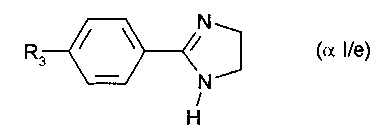 dans laquelle R₃ représente un radical choisi parmi un radical éthyle, n-propyle, n-butyle, trifluorométhyle, (C₁-C₆) alkylthio, trifluorométhoxy, phényle, phénoxy, (C₁-C₆) acylamino, aminosulfonyle, aminosulfonyle substitué sur l'atome d'azote par un ou deux groupements (C₁-C₆) alkyle linéaire ou ramifié,
leurs isomères optiques, ainsi que leurs sels d'addition à un acide pharmaceutiquement acceptable.

7. Composés selon la revendication 1 de formule (α I/f): 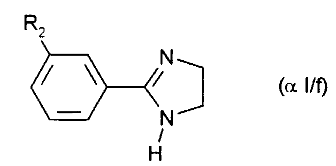 dans laquelle R₂ représente un atome d'halogène différent d'un chlore et d'un fluor ou un groupement (C₂-C₆) alkyle linéaire ou ramifié ou (C₁-C₆) alkylcarbonyle linéaire ou ramifié.
leurs isomères optiques, ainsi que leurs sels d'addition à un acide pharmaceutiquement acceptable.

8. Composés de formule (I) selon la revendication 1 pour lesquels R représente un radical β 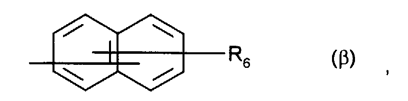 dans lequel R₆ représente un atome d'halogène, un groupement (C₁-C₆) alkyle linéaire ou ramifié, ou méthoxy,
leurs isomères optiques, ainsi que leurs sels d'addition à un acide pharmaceutiquement acceptable.

9. Composé selon la revendication 1 qui est le 2-(4-biphényl)Δ-2 imidazoline ainsi que ses sels d'addition à un acide pharmaceutiquement acceptable.

10. Composé selon la revendication 1 qui est le 2-(3-cyano-2-méthylphényl)Δ-2 imidazoline ainsi que ses sels d'addition à un acide pharmaceutiquement acceptable.

11. Composé selon la revendication 1 qui est le 2-(4-phénoxyphényl)Δ-2 imidazoline ainsi que ses sels d'addition à un acide pharmaceutiquement acceptable.

12. Composé selon la revendication 1 qui est le 2-(4-éthylphényl)Δ-2 imidazoline ainsi que ses sels d'addition à un acide pharmaceutiquement acceptable.

13. Procédé de préparation des composés de formule (I) selon la revendication 1, caractérisé en ce que l'on opère une condensation d'un nitrile de formule (II)
R-C≡N (II)
dans laquelle R est tel que défini précédemment,
avec l'éthylène diamine, en présence d'une quantité catalytique de pentasulfure de phosphore,
le composé brut ainsi obtenu pouvant être, si on le désire :
- purifié suivant une ou plusieurs méthodes de purification choisies parmi la cristallisation, la chromatographie sur gel de silice, l'extraction, la filtration et le passage sur charbon ou sur résine,
- séparé, le cas échéant, sous forme pure ou sous forme de mélange, en ses éventuels isomères optiques, selon des techniques classiques de séparation,
- et/ou transformé par un acide, en sels pharmaceutiquement acceptables.

14. Compositions pharmaceutiques contenant les composés de formule (I) selon l'une des revendications 1 à 12 ou leurs sels d'addition à un acide, pharmaceutiquement acceptables, en combinaison avec un ou plusieurs excipients pharmaceutiquement acceptables.

15. Compositions pharmaceutiques selon la revendication 14 utiles pour le traitement des pathologies liées aux récepteurs aux imidazolines.

16. Compositions pharmaceutiques selon l'une des revendications 14 ou 15, utiles dans le traitement des pathologies liées au système nerveux central, de la dépression, de la maladie de Parkinson, de l'anorexie, des pathologies cardio-vasculaires, de l'hypertension, du diabète, de l'obésité, de l'anémie et du cancer.

## Patentansprüche

1. Verbindungen der Formel (I): 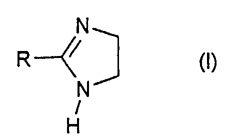 in der R:
• entweder eine Phenylgruppe der Formel (α): 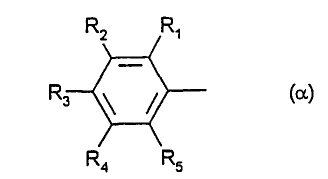 in der R₁:
◆ entweder eine (C₁-C₆)-Alkylgruppe und in diesem Fall:
◇ entweder R₂ ein Wasserstoffatom ebenso wie R₃ und R₅ und in diesem Fall R₄ ein von Chlor verschiedenes Halogenatom darstellen,
◇ oder R₂ ein Wasserstoffatom ebenso wie R₄ und R₅ und in diesem Fall R₃ ein von Chlor verschiedenes Halogenatom darstellen,
◇ oder R₂ eine Cyanogruppe und R₃, R₄ und R₅ jeweils ein Wasserstoffatom darstellen,
◆ oder ein Halogenatom und in diesem Fall:
◇ entweder R₃ ebenfalls ein von R₁ verschiedenes Halogenatom und R₂, R₄ und R₅ jeweils ein Wasserstoffatom darstellen,
◇ oder R₃ ein Wasserstoffatom ebenso wie R₂ und R₅ und R₄ dann eine (C₁-C₆)-Alkylgruppe darstellen,
◆ oder ein Wasserstoffatom und in diesem Fall:
◇ entweder R₂, R₃ und R₄ gleichzeitig ein Wasserstoffatom und R₅ eine Phenylgruppe darstellen,
◇ oder R₂ und R₃ gleichzeitig voneinander verschiedene Halogenatome und R₄ und R₅ jeweils ein Wasserstoffatom darstellen,
◇ oder R₂ ein von Chlor und Fluor verschiedenes Halogenatom oder eine (C₂-C₆)-Alkylgruppe oder (C₁-C₆)-Alkylcarbonylgruppe und R₃, R₄ und R₅ jeweils ein Wasserstoffatom darstellen,
◇ oder R₂ ein Wasserstoffatom ebenso wie R₄und R₅ darstellen und in diesem Fall R₃ aus Ethyl-, n-Propyl-, n-Butyl-, Trifluormethyl-, (C₁-C₆)-Alkylthio-, Trifluormethoxy-, Phenyl-, Phenoxy-, (C₁-C₆)-Acylamino-, Aminosulfonyl- oder am Stickstoffatom durch eine oder zwei (C₁-C₆)-Alkylgruppen substituierte Aminosulfonylgruppen ausgewählt ist,
◇ oder R₂ eine Nitrogruppe, R₃ eine Hydroxygruppe, R₄ ein Halogenatom und R₅ ein Wasserstoffatom darstellen,
◇ oder R₃ und R₅ Jewells ein Wasserstoffatom und R₂ und R₄ jeweils ein Halogenatom ohne gleichzeitig zwei Chlor zu bedeuten, darstellen.
• oder eine Naphthylgruppe der Formel (β): 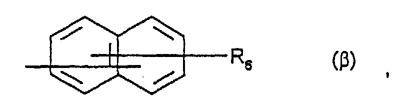 in der R₆ ein Halogenatom, eine (C₁-C₆)-Alkylgruppe oder eine Methoxygruppe darstellt,
• oder eine Gruppe ausgewählt aus den folgenden Gruppen:
- Indol-5-yl 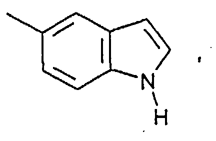
- 1-Phenyl-1-cyclohexylmethyl,
- Cycloheptyl,
- 4-(Benzothiazol-2-yl)-benzyl und
- die Gruppe 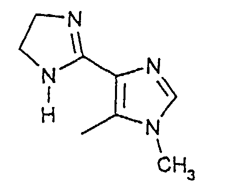
bedeuten,
deren optische Isomeren sowie deren Additionssalze mit einer pharmazeutisch annehmbaren Säure,
wobei es sich versteht, daß unter (C₁-C₆)-Alkylgruppen, (C₂-C₆)-Alkylgruppen und (C₁-C₆)-Alkylthiogruppen sowohl geradkettige als auch verzweigte Gruppen zu verstehen sind.

2. Verbindungen nach Anspruch 1 der Formel (α I/a): 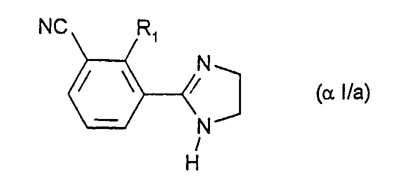 in der R₁ eine geradkettige oder verzweigte (C₁-C₆)-Alkylgruppe darstellt, deren optische Isomeren sowie deren Additionssalze mit einer pharmazeutisch annehmbaren Säure.

3. Verbindungen nach Anspruch 1 der Formel (α I/b): 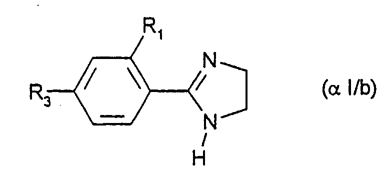 in der R₁ ein Halogenatom oder eine geradkettige oder verzweigte (C₁-C₆)-Alkylgruppe und R₃, welches von R₁ verschieden ist, ein Halogenatom bedeuten (wobei R₃ von einem Chloratom verschieden ist, wenn R₁ eine geradkettige oder verzweigte (C₁-C₆)-Alkylgruppe darstellt),
deren optische Isomeren sowie deren Additionssalze mit einer pharmazeutisch annehmbaren Säure.

4. Verbindungen nach Anspruch 1 der Formel (α I/c): 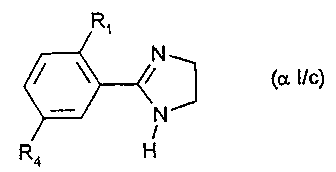 in der R₁ ein Halogenatom und R₄ eine geradkettige oder verzweigte (C₁-C₆)-Alkylgruppe bedeuten,
deren optische Isomeren sowie deren Additionssalze mit einer pharmazeutisch annehmbaren Säure.

5. Verbindungen nach Anspruch 1 der Formel (α I/d): 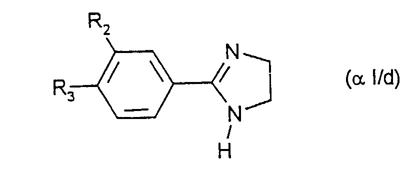 in der R₂ und R₃ jeweils ein voneinander verschiedenes Halogenatom bedeuten,
sowie deren Additionssalze mit einer pharmazeutisch annehmbaren Säure.

6. Verbindungen nach Anspruch 1 der Formel (α I/e): 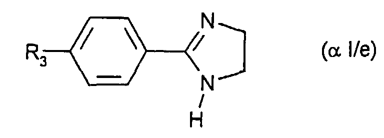 in der R₃ eine Gruppe ausgewählt aus Ethyl-, n-Propyl-, n-Butyl-, Trifluormethyl-, (C₁-C₆)-Alkylthio-, Trifluormethoxy-, Phenyl-, Phenoxy-, (C₁-C₆)-Acylamino-, Aminosulfonyl- und am Stickstoffatom durch eine oder zwei geradkettige oder verzweigte (C₁-C₆)-Alkylgruppen substituierte Aminosulfonylgruppen ausgewählte Gruppe bedeutet,
deren optische Isomeren sowie deren Additionssalze mit einer pharmazeutisch annehbmaren Säure.

7. Verbindungen nach Anspruch 1 der Formel (α I/f): 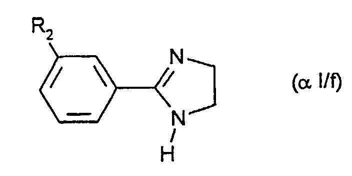 in der R₂ ein von Chlor und Fluor verschiedenes Halogenatom oder eine geradkettige oder verzweigte (C₂-C₆)-Alkylgruppe oder eine geradkettige oder verzweigte (C₁-C₆)-Alkylcarbonylgruppe bedeutet,
deren optische Isomeren sowie deren Additionssalze mit einer pharmazeutisch annehmbaren Säure.

8. Verbindungen der Formel (I) nach Anspruch 1, in der R eine Gruppe darstellt: 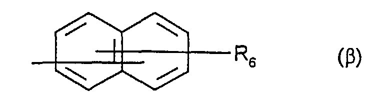 in der R₆ ein Halogenatom, eine geradkettige oder verzweigte (C₁-C₆)-Alkylgruppe oder eine Methoxygruppe bedeutet,
deren optische Isomeren sowie deren Additionssalze mit einer pharmazeutisch annehmbaren Säure.

9. Verbindung nach Anspruch 1, nämlich 2-(4-Biphenyl)-Δ-2-imidazolin sowie dessen Additionssalze mit einer pharmazeutisch annehmbaren Säure.

10. Verbindung nach Anspruch 1, nämlich 2-(3-Cyano-2-methylphenyl)-Δ-2-imidazolin sowie dessen Additionssalze mit einer pharmazeutisch annehmbaren Säure.

11. Verbindung nach Anspruch 1, nämlich 2-(4-Phenoxyphenyl)-Δ-2-imidazolin sowie dessen Additionssalze mit einer pharmazeutisch annehmbaren Säure.

12. Verbindung nach Anspruch 1, nämlich 2-(4-Ethylphenyl)-Δ-2-imidazolin sowie dessen Additionssalze mit einer pharmazeutisch annehmbaren Säure.

13. Verfahren zur Herstellung der Verbindungen der Formel (I) nach Anspruch 1, dadurch gekennzeichnet, daß man ein Nitril der Formel (II):
R - C ≡ N (II)
in der R die oben angegebenen Bedeutungen besitzt, mit Ethylendiamin in Gegenwart einer katalytischen Menge von Phosphorpentasulfid kondensiert,
wobei die in dieser Weise erhaltene rohe Verbindung gewünschtenfalls:
- gemäß eines oder mehrerer Reinigungsverfahren ausgewählt aus Kristallisation, Chromatographie über Kieselgel, Extraktion, Filtration und Überführung über Aktivkohle oder ein Harz gereinigt werden kann,
- gegebenenfalls gemäß klassischer Trennverfahren in ihre eventuellen optischen Isomeren in reiner Form oder in Form einer Mischung getrennt werden kann,
- und/oder mit einer Säure in pharmazeutisch annehmbare Salze umgewandelt werden kann.

14. Pharmazeutische Zubereitungen enthaltend die Verbindungen der Formel (I) nach einem der Ansprüche 1 bis 12 oder ihre Additionssalze mit einer pharmazeutisch annehmbaren Säure in Kombination mit einem oder mehreren pharmazeutisch annehmbaren Trägermaterialien.

15. Pharmazeutische Zubereitungen nach Anspruch 14 zur Behandlung von pathologischen Zuständen, die mit Imidazolin-Rezeptoren verknüpft sind.

16. Pharmazeutische Zubereitungen nach einem der Ansprüche 14 oder 15 zur Behandlung von pathologischen Zuständen, die das Zentralnervensystem, Depressionen, die Parkinsonsche Krankheit, Anorexie, kardiovaskuläre pathologische Zustände, Hypertension, Diabetes, Fettsucht, Anämie und Krebs betreffen.

## Claims

1. Compounds of formula (I): 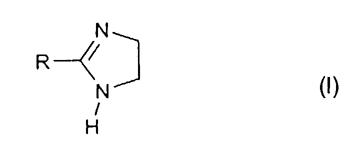 wherein R represents:
• a phenyl radical of formula (α): 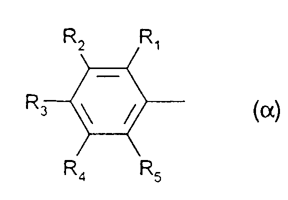 wherein R₁ represents:
◆ a (C₁-C₆)alkyl radical, in which case:
◇ R₂ represents a hydrogen atom as do R₃ and R₅, in which case R₄ represents a halogen atom other than chlorine,
◇ R₂ represents a hydrogen atom as do R₄ and R₅, in which case R₃ represents a halogen atom other than chlorine, or
◇ R₂ represents the cyano radical, and R₃, R₄ and R₅ each represent a hydrogen atom,
◆ a halogen atom, in which case:
◇ R₃ also represents a halogen atom, other than R₁, and R₂, R₄ and R₅ each represent a hydrogen atom, or
◇ R₃ represents a hydrogen atom as do R₂ and R₅, and R₄ represents a (C₁-C₆)alkyl radical,
or
◆ a hydrogen atom, in which case:
◇ R₂, R₃ and R₄ simultaneously represent a hydrogen atom and R₅ represents a phenyl group,
◇ R₂ and R₃ simultaneously represent a halogen atom each different from the other, and R₄ and R₅ each represent a hydrogen atom,
◇ R₂ represents a halogen atom other than chlorine and fluorine, or a (C₂-C₆)alkyl or (C₁-C₆)alkylcarbonyl group, and R₃, R₄ and R₅ each represent a hydrogen atom,
◇ R₂ represents a hydrogen atom as do R₄ and R₅, in which case R₃ is selected from the radicals: ethyl, n-propyl, n-butyl, trifluoromethyl, (C₁-C₆)alkylthio, trifluoromethoxy, phenyl, phenoxy, (C₁-C₆)acylamino, aminosulphonyl and aminosulphonyl substituted on the nitrogen atom by one or two (C₁-C₆)alkyl groups,
◇ R₂ represents a nitro group, R₃ represents a hydroxy group, R₄ represents a halogen atom and R₅ represents a hydrogen atom, or
◇ R₃ and R₅ each represent a hydrogen atom, and R₂ and R₄ each represent a halogen atom but cannot simultaneously represent two chlorine atoms,
• a naphthyl radical of formula (β): 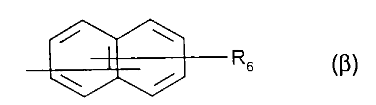 wherein R₆ represents a halogen atom, a (C₁-C₆)alkyl group or methoxy,
or
• a radical selected from the radicals
- indol-5-yl 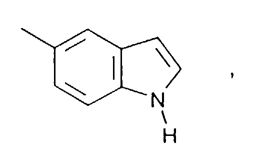
- 1-phenyl-1-cyclohexylmethyl,
- cycloheptyl,
- 4-(benzothiazol-2-yl)benzyl, and
- the radical 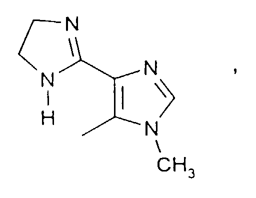
their optical isomers, and pharmaceutically acceptable addition salts thereof with an acid,
it being understood that the radicals (C₁-C₆)alkyl, (C₂-C₆)alkyl and (C₁-C₆)alkylthio are understood to mean both the linear and branched radicals.

2. Compounds according to claim 1 of formula (α I/a): 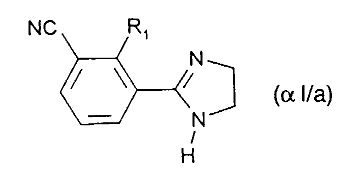 wherein R₁ represents a linear or branched (C₁-C₆)alkyl group,
their optical isomers, and addition salts thereof with a pharmaceutically acceptable acid.

3. Compounds according to claim 1 of formula (α I/b): 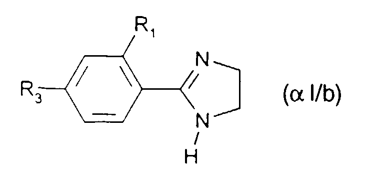 wherein R₁ represents a halogen atom or a linear or branched (C₁-C₆)alkyl group, and R₃, which is other than R₁, represents a halogen atom (R₃ being other than a chlorine atom when R₁ represents a linear or branched (C₁-C₆)-alkyl group),
their optical isomers, and addition salts thereof with a pharmaceutically acceptable acid.

4. Compounds according to claim 1 of formula (α I/c): 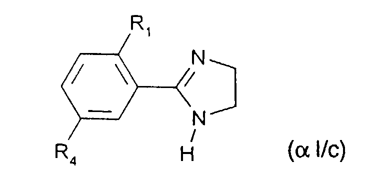 wherein R₁ represents a halogen atom and R₄ represents a linear or branched (C₁-C₆)alkyl radical,
their optical isomers, and addition salts thereof with a pharmaceutically acceptable acid.

5. Compounds according to claim 1 of formula (α I/d): 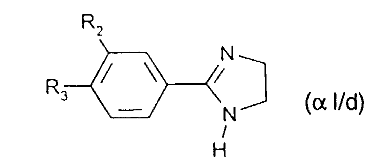 wherein R₂ and R₃ each represent a halogen atom each different from the other,
and addition salts thereof with a pharmaceutically acceptable acid.

6. Compounds according to claim 1 of formula (α I/e): 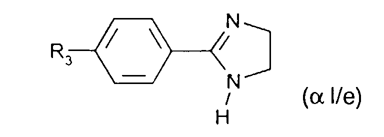 wherein R₃ represents a radical selected from the radicals: ethyl, n-propyl, n-butyl, trifluoromethyl, (C₁-C₆)alkylthio, trifluoromethoxy, phenyl, phenoxy, (C₁-C₆)acylamino, aminosulphonyl and aminosulphonyl substituted on the nitrogen atom by one or two linear or branched (C₁-C₆)alkyl groups,
their optical isomers, and addition salts thereof with a pharmaceutically acceptable acid.

7. Compounds according to claim 1 of formula (α I/f): 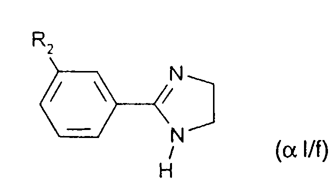 wherein R₂ represents a halogen atom other than chlorine and fluorine, or a linear or branched (C₂-C₆)alkyl or linear or branched (C₁-C₆)alkylcarbonyl group,
their optical isomers, and addition salts thereof with a pharmaceutically acceptable acid.

8. Compounds of formula (I) according to claim 1 wherein R represents a radical β 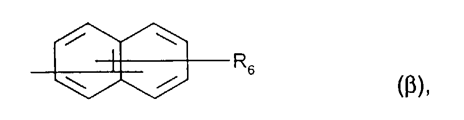 wherein R₆ represents a halogen atom, a linear or branched (C₁-C₆)alkyl group, or methoxy,
their optical isomers, and addition salts thereof with a pharmaceutically acceptable acid.

9. Compound according to claim 1 which is 2-(4-biphenyl)-Δ²-imidazoline and its addition salts with a pharmaceutically acceptable acid.

10. Compound according to claim 1 which is 2-(3-cyano-2-methylphenyl)-Δ²-imidazoline and its addition salts with a pharmaceutically acceptable acid.

11. Compound according to claim 1 which is 2-(4-phenoxyphenyl)-Δ²-imidazoline and its addition salts with a pharmaceutically acceptable acid.

12. Compound according to claim 1 which is 2-(4-ethylphenyl)-Δ²-imidazoline and its addition salts with a pharmaceutically acceptable acid.

13. Process for the preparation of compounds of formula (I) according to claim 1, characterised in that a nitrile of formula (II):
R-C≡N (II)
wherein R is as defined hereinabove,
is condensed with ethylenediamine in the presence of a catalytic amount of phosphorus pentasulphide,
it being possible for the resulting crude product, if desired, to be:
- purified according to one or more purification methods selected from crystallisation, chromatography over silica gel, extraction, filtration and passage over carbon or resin,
- separated, where appropriate, in pure form or in the form of a mixture, into its possible optical isomers, according to conventional separation techniques, and/or
- converted by an acid into pharmaceutically acceptable salts.

14. Pharmaceutical compositions comprising the compounds of formula (I) according to any one of claims 1 to 12 or pharmaceutically acceptable addition salts thereof with an acid, in combination with one or more pharmaceutically acceptable excipients.

15. Pharmaceutical compositions according to claim 14 for use in the treatment of pathologies associated with imidazoline receptors.

16. Pharmaceutical compositions according to any one of claims 14 or 15, for use in the treatment of pathologies associated with the central nervous system, depression, Parkinson's disease, anorexia, cardiovascular pathologies, hypertension, diabetes, obesity, anaemia and cancer.
